# EUROPEAN PATENT APPLICATION

(11) **EP 2 123 208 A2**
(43) Date of publication of application: **25.11.2009**
(21) Application number: 09006540.0
(22) Date of filing: 14.05.2009
(51) Int. Cl.: A61B 1/00, A61B 1/045

(54) **Endoscope system with option circuit board**

(30) Priority: 20.05.2008 JP 2008131927
(71) Applicant: FUJIFILM Corporation, Minato-ku Tokyo (JP)
(72) Inventor: Abe, Kazunori, Saitama-shi Saitama (JP)
(74) Representative: Klunker . Schmitt-Nilson . Hirsch

(57) **Abstract**

An endoscope system (2) has an electronic endoscope (10) with a solid-state image sensor (40), a processor device (11, 91), and an option circuit board (56, 92) detachably connected to the processor device. The electronic endoscope drives the solid-state image sensor and captures images. The processor device generates a first video signal from an image signal. The option circuit board has a measurement pulse generator (81) for generating measurement pulses, a gate pulse generator (82) for generating a gate pulse, a pulse counter (83) for counting the number of the measurement pulses during outputting the gate pulse, and a judgment circuit (84) for obtaining the number of pixels from a count value of the pulse counter. The option circuit board converts the first video signal into a second video signal in consideration of the number of pixels to display the images on a PC monitor (87).

## Description

### BACKGROUND OF THE INVENTION

### 1. Field of the Invention

The present invention relates to a detachable option circuit board having the function of converting the format of a video signal, and an endoscope system with the option circuit board.

### 2. Description Related to the Prior Art

In recent years, an endoscope system is widely used in medical examinations. The endoscope system is constituted of an electronic endoscope with a solid-state image sensor and a processor device to which the electronic endoscope is detachably connected. The processor device controls the actuation of the solid-state image sensor contained in the electronic endoscope, and produces images (endoscope images) from an image signal outputted from the solid-state image sensor. There are various types of electronic endoscopes according to difference in the number of pixels of the solid-state image sensor. Is known a processor device to which the electronic endoscopes with various numbers of pixels are connectable (refer to, for example, Japanese Patent Laid-Open Publication No. 2005-296534).

Generally, the processor device converts the image signal from the solid-state image sensor into an NTSC format being a standard television picture format, and displays the endoscope images on a TV monitor. There are some cases of displaying the endoscope images on a PC monitor recently, but every user does not always require such a display mode. Thus, US Patent No. 7,429,242 (corresponding to Japanese Patent Laid-Open Publication No. 2003-24272) discloses to prepare an interface circuit that converts the image signal into a PC monitor-compatible format as an option circuit board (video card) separately from the processor device. The option circuit board is connected to the processor device as necessary.

However, when the interface circuit is provided in the option circuit board, as described above, the option circuit board communicates with a CPU in the processor device to obtain the number of pixels (resolution) of the solid-state image sensor from the CPU. Considering the number of pixels, are produced images to display on the PC monitor. Accordingly, it takes relatively long time between turning power on and the PC monitor starting displaying the images through the option circuit board, resulting in inconvenience for the users.

### SUMMARY OF THE INVENTION

An object of the present invention is to provide an option circuit board that shortens image output processing time.

Another object of the present invention is to provide an endoscope system that shortens waiting time for outputting endoscope images by using the option circuit board.

To achieve the foregoing objects, an option circuit board according to the present invention is provided with a measurement pulse generator, a measurement period setting circuit, a pulse counter, a judgment circuit, and a signal processing circuit. The option circuit board is detachably connected to a processor device. The processor device is connected to an electronic endoscope with a solid-state image sensor. The solid-state image sensor is driven by a clock signal whose frequency corresponds to the number of pixels. The solid-state image sensor captures an image in a human body cavity, and generates an image signal. The processor device produces a first video signal from the image signal.

The measurement pulse generator generates measurement pulses at constant intervals. The measurement period setting circuit sets a period of time during a predetermined number of clocks of the clock signal as a measurement period. The pulse counter counts the number of measurement pulses generated by the measurement pulse generator during the measurement period. The judgment circuit judges the number of pixels from a count value of the pulse counter. The signal processing circuit converts the first video signal from the processor device into a second video signal in consideration of the number of pixels to display the image on external equipment.

The external equipment may be a PC monitor. The judgment circuit is provided with a memory that stores data indicating relation between the count value and the number of pixels, and refers to the data in the memory to find out the number of pixels. The signal processing circuit has a synchronizing signal generator and a DVI circuit. The synchronizing signal generator generates a synchronizing signal for the second video signal, and inputs the synchronizing signal to the DVI circuit. The DVI circuit outputs a DVI-format digital video signal as the second video signal that is produced from the first video signal and the synchronizing signal.

The synchronizing signal generator corrects deviation in phase between a first horizontal synchronizing signal and a first vertical synchronizing signal, which are outputted from the processor device, and then outputs a second horizontal synchronizing signal and a second vertical synchronizing signal as the synchronizing signals.

The first video signal includes a luminance signal and a color-difference signal. The synchronizing signal generator includes an OR circuit and a flip-flop. To the OR circuit, the luminance signal and the color-difference signal are inputted. The flip-flop has a data input terminal, a clock signal input terminal, and an output terminal. To the data input terminal, an output signal of the OR circuit is inputted. To the clock signal input terminal, the clock signal is inputted. The output terminal outputs the output signal of the OR circuit as the synchronizing signal in synchronization with the clock signal.

An endoscope system according to the present invention includes an electronic endoscope having a solid-state image sensor, a processor device connected to the electronic endoscope, and an option circuit board detachably connected to the processor device. The solid-state image sensor is driven by a clock signal whose frequency corresponds to the number of pixels. The solid-state image sensor captures an image in a human body cavity and generates an image signal. The processor device is provided with a first signal processing circuit that produces a first video signal from the image signal. The first video signal is sent to an NTSC-format monitor connected to the processor device. The option circuit board is provided with a pixel number judgment section and a second signal processing circuit. The pixel number judgment section obtains the number of pixels from the frequency of the clock signal. The second signal processing circuit converts the first video signal into a second video signal in consideration of the number of pixels to display the image on external equipment.

According to the present invention, since the option circuit board can judge the number of pixels (resolution) of the solid-state image sensor based on the clock signal of the processor device, it becomes unnecessary to obtain the number of pixels from the processor device. Thus, image format conversion is quickly performed, and hence it is possible to shorten waiting time for start displaying the image on the external equipment.

### BRIEF DESCRIPTION OF THE DRAWINGS

For more complete understanding of the present invention, and the advantage thereof, reference is now made to the following descriptions taken in conjunction with the accompanying drawings, in which:
Fig. 1 is a schematic perspective view of an endoscope system;
Fig. 2 is a front view of a distal portion of an electronic endoscope;
Fig. 3 is a block diagram of an endoscope system according to a first embodiment;
Fig. 4 is a block diagram of an option circuit board according to the first embodiment;
Fig. 5A is an operation timing chart of the option circuit board on a solid-state image sensor with a small number of pixels;
Fig. 5B is an operation timing chart of the option circuit board on a solid-state image sensor with a large number of pixels;
Fig. 6 is a block diagram of an endoscope system according to a second embodiment;
Fig. 7 is a block diagram of an option circuit board according to the second embodiment;
Fig. 8 is a block diagram of a synchronizing signal generator provided in the option circuit board of the second embodiment; and
Fig. 9 is a timing chart of the synchronizing signal generator shown in Fig. 8.

### DESCRIPTION OF THE PREFERRED EMBODIMENTS

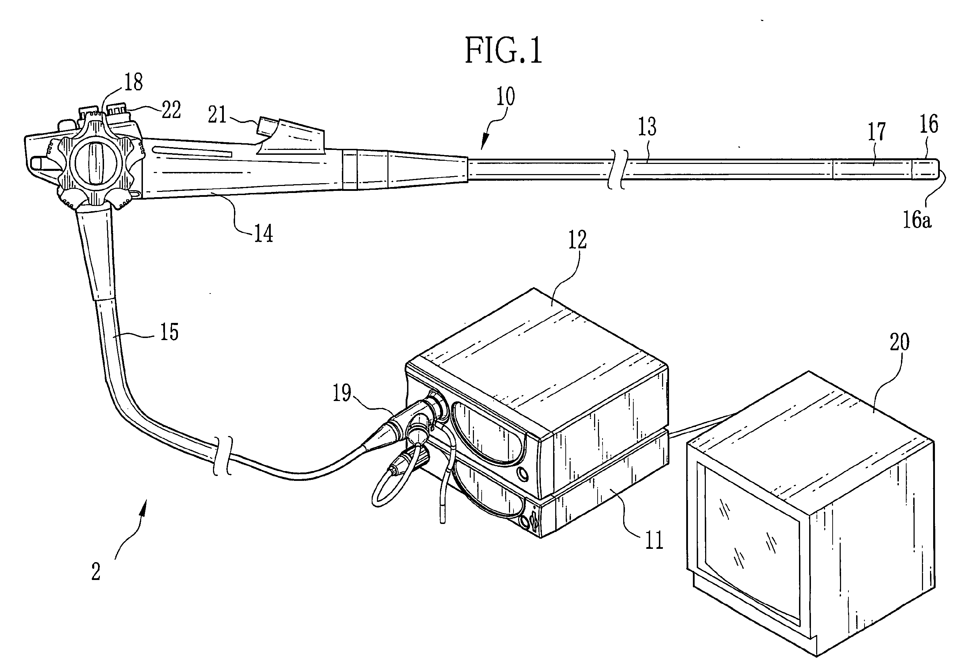

In Fig. 1, an endoscope system 2 is constituted of an electronic endoscope 10, a processor device 11, and a light source device 12. The electronic endoscope 10 is provided with a flexible insert section 13 that is introduced into a human body cavity, an operation section 14 joined to a base end of the insert section 13, and a universal cord 15 connected to the processor device 11 and the light source device 12.

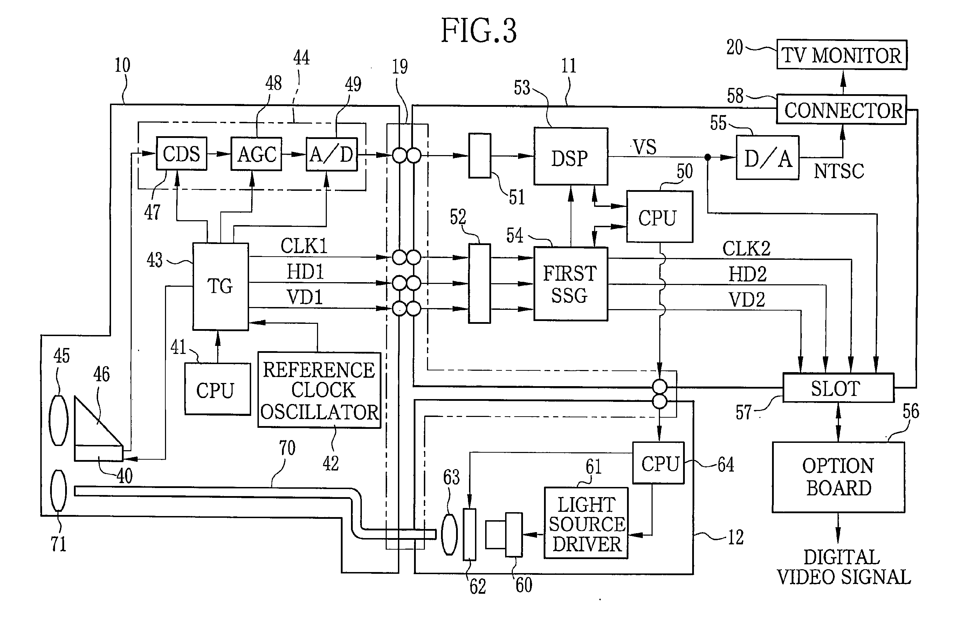

At an end of the insert section 13 is provided a distal portion 16 that contains a solid-state image sensor 40 (refer to Fig. 3) for capturing images inside the human body cavity. Behind the distal portion 16, a bending portion 17, which consists of a number of linked articulated segments, is provided. By operating an angle knob 18 on the operation section 14, a number of wires extending in the insert section 13 are pulled and pushed to bend the bending portion 17 from side to side and up and down. Thus, the distal portion 16 is directed to a desired direction inside the human body cavity.

To an end of the universal cord 15, a multi-connector 19 is attached. The electronic endoscope 10 is detachably connected to the processor device 11 and the light source device 12 via the connector 19. The processor device 11 is electrically connected to the light source device 12 via the connector 19, and centrally controls the actuation of the electronic endoscope 10 and the light source device 12.

The processor device 11 feeds electric power to the electronic endoscope 10 through a transmission cable extending in the universal cord 15, and controls the actuation of the solid-state image sensor 40. The processor device 11 receives an image signal outputted from the solid-state image sensor 40. The processor device 11 applies various kinds of signal processing to the received image signal, and produces image data (first video signal). The produced image data is displayed as endoscope images on an NTSC-format TV monitor 20 connected to the processor device 11 with a cable and/or on a PC monitor 87 connected through an option circuit board 56 (refer to Fig. 3) described later.

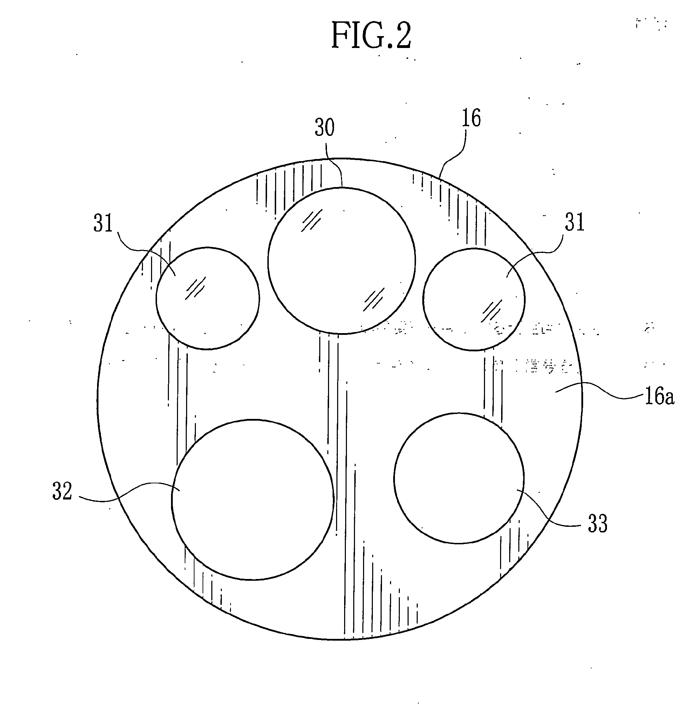

As shown in Fig. 2, a front face 16a of the distal portion 16 is provided with an image capturing window 30, lighting windows 31, a medical instrument outlet 32, and an airing/watering nozzle 33. The image capturing window 30 is disposed in the upper middle of the front face 16a. Behind the image capturing window 30, the solid-state image sensor 40 is disposed through an objective optical system 45 and a prism 46 (refer to Fig. 3).

The two lighting windows 31 are symmetric with respect to the image capturing window 30. Through the lighting windows 31, light being guided from the light source device 12 via a light guide 70 and a lens 71 (refer to Fig. 3) illuminates the inside of the human body cavity. The medical instrument outlet 32 is coupled to a medical instrument insertion port 21 (refer to Fig. 1) provided on the operation section 14 through a channel extending in the insert section 13. Various kinds of medical instruments having a forceps, a needle, a diathermy knife, and the like at their tips are inserted into the medical instrument insertion port 21 in order to protrude the tip of the instrument from the medical instrument outlet 32 in the human body cavity.

The watering/airing nozzle 33 ejects water or air from a water reservoir or an air reservoir contained in the light source device 12 to the image capturing window 30 or a target body part in response to the operation of a watering/airing button 22 (refer to Fig. 1) on the operation section 14.

Referring to Fig. 3, the electronic endoscope 10 has the solid-state image sensor 40 disposed in the distal portion 16. The electronic endoscope 10 also has a CPU 41, a reference clock oscillator 42, a timing generator (TG) 43, and an analog front end processor (AFE) 44 that are disposed in the operation section 14. The solid-state image sensor 40 such as a CCD image sensor is so disposed that object light, which has passed through the objective optical system 45 and the prism 46, is incident upon its light receiving surface. The light receiving surface is equipped with a color filter having a plurality of color segments (for example, primary-colors filter of Bayer arrangement).

The CPU 41 controls actuation of the electronic endoscope 10. The TG 43 generates drive pulses (vertical and horizontal scan pulses, a reset pulse, and the like) based on a reference clock signal from the reference clock oscillator 42, and drives the solid-state image sensor 40. The TG 43 also generates a synchronizing signal for the AFE 44 and inputs the synchronizing signal to the AFE 44. The solid-state image sensor 40 captures images inside the human body cavity in response to the drive pulses from the TG 43, and outputs an image signal.

The number of pixels of the solid-state image sensor 40 varies from model to model of the electronic endoscope 10. The clock frequency of the reference clock oscillator 42 is determined in accordance with the number of pixels of the solid-state image sensor 40 so that the image signal of a constant number of frames (for example, 60 frames) is outputted per second even if the solid-state image sensor 40 has the different number of pixels.

The AFE 44 is constituted of a correlated double sampling circuit (CDS) 47, an automatic gain controller (AGC) 48, and an analog-to-digital converter (A/D) 49. The CDS 47 applies correlated double sampling processing to the image signal outputted from the solid-state image sensor 40 in order to remove reset noise and amplifier noise. The AGC 48 amplifies the image signal without noise by designated gain. The A/D 49 converts the amplified image signal into a digital signal of a predetermined number of bits, and inputs the digital signal to the processor device 11 through the connector 19.

The TG 43 generates a first horizontal synchronizing signal "HD1", a first vertical synchronizing signal "VD1", and a first clock signal "CLK1", and inputs the signals "HD1", "VD1", and "CLK1" to the processor device 11 through the connector 19. These signals "HD1", "VD1", and "CLK1" synchronize with the image signal outputted from the AFE 44.

The processor device 11 includes a CPU 50, isolation devices (IDs) 51 and 52, a digital signal processor (DSP) 53, a first synchronizing signal generator (first SSG) 54, a digital-to-analog converter (D/A) 55, a slot 57, and a connector 58. The CPU 50 controls individual circuits in the processor device 11 and the actuation of the light source device 12. The IDs 51 and 52 electrically separate the electronic endoscope 10 from the processor device 11. An option circuit board 56 is connected to the slot 57, and the TV monitor 20 is connected to the connector 58.

To the first SSG 54, the first horizontal synchronizing signal "HD1", the first vertical synchronizing signal "VD1", and the first clock signal "CLK1" are inputted through the ID 52. The first SSG 54 corrects deviation in phase among the first horizontal synchronizing signal "HD1", the first vertical synchronizing signal "VD1", and the first clock signal "CLK1", and produces a second horizontal synchronizing signal "HD2", a second vertical synchronizing signal "VD2", and a second clock signal "CLK2". The first SSG 54 inputs these signals "HD2", "VD2", and "CLK2" to the option circuit board 56 through the DSP 53 and the slot 57.

To the DSP 53, the image signal outputted from the AFE 44 of the electronic endoscope 10 is inputted through the ID 51. The DSP 53 subjects the image signal to image processing such as color separation, color interpolation, gain correction, white balance correction, gamma correction, and image enhancement in order to generate a YC-format image data (video signal) "VS". The video signal "VS" consists of a luminance (Y) signal and a color difference (C) signal. The DSP 53 inputs the video signal "VS" to the D/A 55 and the option circuit board 56 through the slot 57.

The D/A 55 converts the video signal "VS" into an NTSC-format analog video signal "NTSC", and outputs the analog video signal "NTSC" to the TV monitor 20 connected to the connector 58.

The light source device 12 is constituted of a light source 60 such as a xenon lamp and a halogen lamp, a light source driver 61 for driving the light source 60, an aperture stop mechanism 62 and a condenser lens 63 disposed between the light source 60 and the light guide 70, and a CPU 64 for controlling the light source driver 61 and the aperture stop mechanism 62 by communicating with the CPU 50 of the processor device 11. The aperture stop mechanism 62 increases or decreases the amount of light incident upon the light guide 70. The condense lens 63 condenses light, which has passed through the aperture stop mechanism 62, and leads the light into an entry of the light guide 70. The light further propagates through the light guide 70, and is applied to the target body part through the lighting windows 31 as described above.

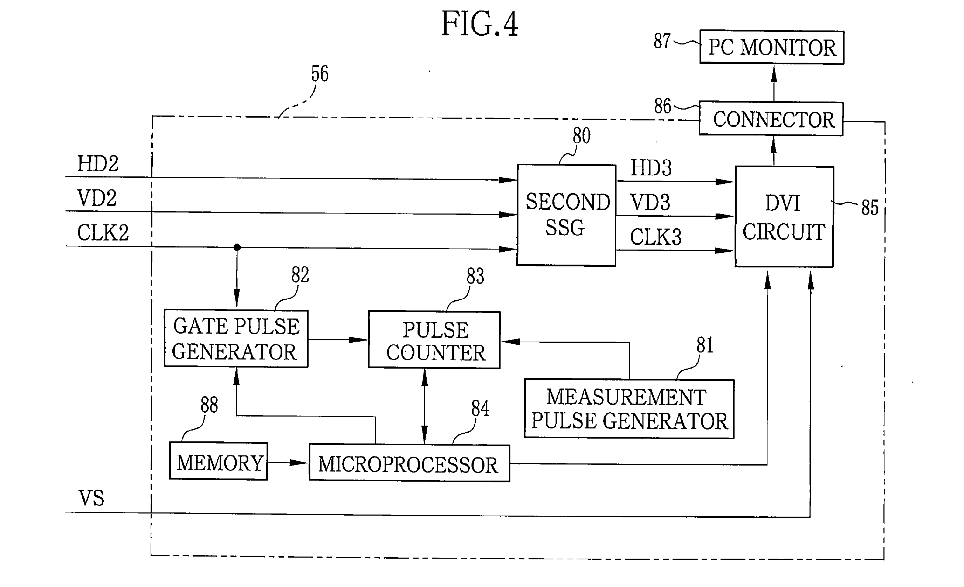

As shown in Fig. 4, the option circuit board 56 is constituted of a second synchronizing signal generator (second SSG) 80, a measurement pulse generator 81, a gate pulse generator (measurement period setting circuit) 82, a pulse counter 83, a microprocessor 84, a digital visual interface (DVI) circuit 85, a connector 86, and a memory 88. The microprocessor 84 and the memory 88 constitute a judgment circuit. The second SSG 80 and the DVI circuit 85 constitute a signal processing circuit.

To the second SSG 80, the second horizontal synchronizing signals "HD2", the second vertical synchronizing signal "VD2", and the second clock signal "CLK2" outputted from the first SSG 54 of the processor device 11 are inputted via the slot 57. The second SSG 80 corrects deviation in phase among the second horizontal synchronizing signal "HD2", the second vertical synchronizing signal "VD2", and the second clock signal "CLK2".
The second SSG 80 outputs the corrected signals as a third horizontal synchronizing signal "HD3", a third vertical synchronizing signal "VD3", and a third clock signal "CLK 3", and inputs these signals "HD3", "VD3", and "CLK3" to the DVI circuit 85.

The measurement pulse generator 81 generates a measurement pulse signal that has higher frequency than the second clock signal "CLK2", and inputs the measurement pulse signal to the pulse counter 83. The gate pulse generator 82 counts a predetermined number of rising edges of the second clock signal "CLK2" (for example, 10 pulses of the second clock signal "CLK2") to generate a gate pulse signal. The gate pulse signal stays at a high level during a counted period and at a low level during a no-counted period. The generated gate pulse signal is inputted to the pulse counter 83.

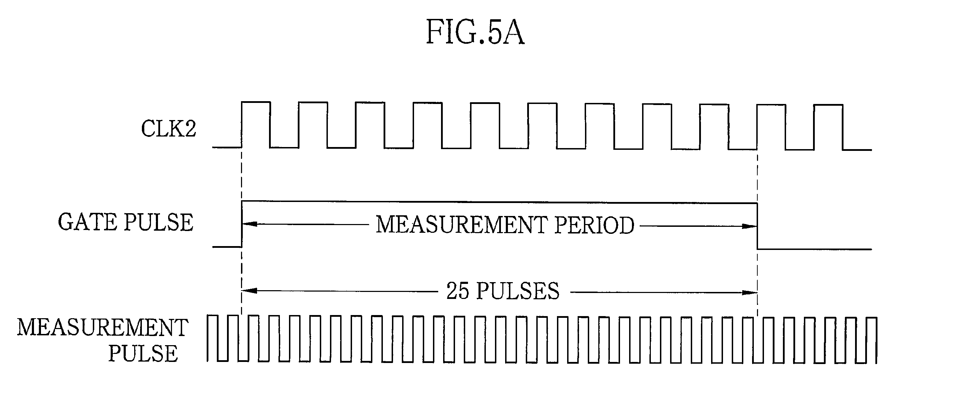

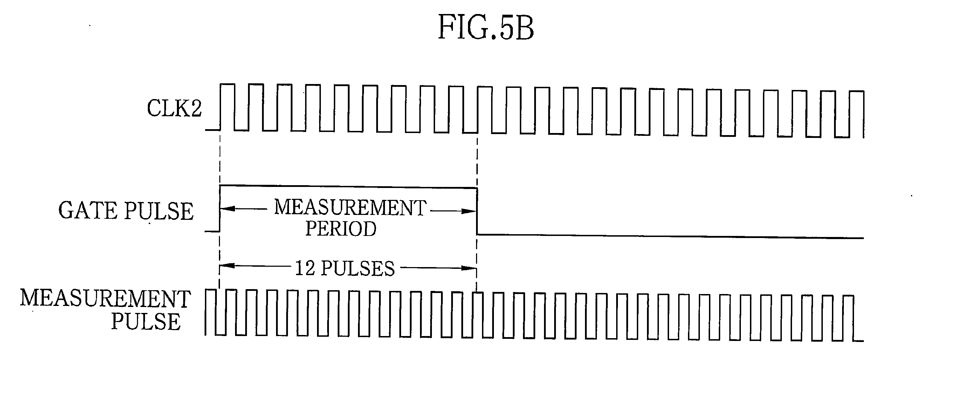

The pulse counter 83 sets a high-level period as a measurement period. The pulse counter 83 counts the number of measurement pulses generated within the measurement period, and inputs a count value to the microprocessor 84. Fig. 5A shows a case where the solid-state image sensor 40 has a small number of pixels (low resolution), and the second clock signal "CLK2" has a low frequency. Fig. 5B shows a case where the solid-state image sensor 40 has a large number of pixels (high resolution), and the frequency of the second clock signal "CLK2" is higher than that of Fig. 5A. In these two examples, the count value of the pulse counter 83 is 25 pulses in Fig. 5A, and is 12 pulses in Fig.5B.

The microprocessor 84 judges the number of pixels (resolution) of the solid-state image sensor 40 based on the count value of the pulse counter 83. The memory 88 stores data that indicates the relation between the count values and the numbers of pixels. The microprocessor 84 retrieves the number of pixels corresponding to the inputted count value from the data stored in the memory 88. For example, when the count value is 24 to 26 pulses, the number of pixels of the solid-state image sensor 40 is judged to be "640×480". When the count value is 12 to 13 pulses, the number of pixels is judged to be "1280 × 960". The microprocessor 84 actuates the gate pulse generator 82 and the pulse counter 83 only during initialization just after turning the processor device 11 on, for the purpose of judging the number of pixels of the solid-state image sensor 40. The number of pixels judged is inputted to the DVI circuit 85.

The DVI circuit 85 determines a display pixel number (display resolution) based on the number of pixels (resolution) of the solid-state image sensor 40 inputted from the microprocessor 84. The DVI circuit 85 also generates a digital video signal for a PC monitor 87 based on the video signal "VS" inputted from the processor device 11 and the third horizontal synchronizing signal "HD3" and the third vertical synchronizing signal "VD3" inputted from the second SSG 80. The DVI circuit 85 outputs the generated digital video signal to the PC monitor 87 that is connected to the option circuit board 56 via the connector 86 to display the endoscope images.

Observing the inside of the human body cavity by using the endoscope system 2, the electronic endoscope 10 is first connected to the processor device 11 and the light source device 12 via the connector 19. Then, the endoscope system 2 is turned on, and the insert section 13 of the electronic endoscope 10 is introduced into the human body cavity. The solid-state image sensor 40 captures the images inside the human body cavity while the light from the light source device 12 illuminates there. The images are displayed on the NTSC-format TV monitor 20 connected to the processor device 11.

When the option circuit board 56 is connected to the slot 57 of the processor device 11, the gate pulse generator 82 and the pulse counter 83 in the option circuit board 56 are actuated in response to turning the processor device 11 on. The gate pulse generator 82 counts a predetermined number of pulses of the inputted second clock signal "CLK2" to generate the gate pulse signal that sets the measurement period. The pulse counter 83 counts the number of measurement pulses generated from the measurement pulse generator 81 during the measurement period, and inputs the count value to the microprocessor 84. The microprocessor 84 judges the number of pixels (resolution) of the solid-state image sensor 40 based on the inputted count value, and inputs the judged number of pixels to the DVI circuit 85. The DVI circuit 85 determines the display resolution on the PC monitor 87 based on the inputted number of pixels. The DVI circuit 85 generates the digital video signal that corresponds to the display resolution. The DVI circuit 85 outputs the digital video signal to the PC monitor 87 through the connector 86, so that the images inside the human body cavity are displayed on the PC monitor 87 too.

As described above, since the option circuit board 56 judges the number of pixels based on the second clock signal "CLK2" inputted from the processor device 11 without communicating with the CPU 50 of the processor device 11, the option circuit board 56 quickly starts producing the digital video signal for the PC monitor 87. Thus, it is possible to reduce time from power-up till the PC monitor 87 starts displaying the images. Also, becomes unnecessary a communication line for communicating the number of pixels between the processor device 11 and the option circuit board 56.

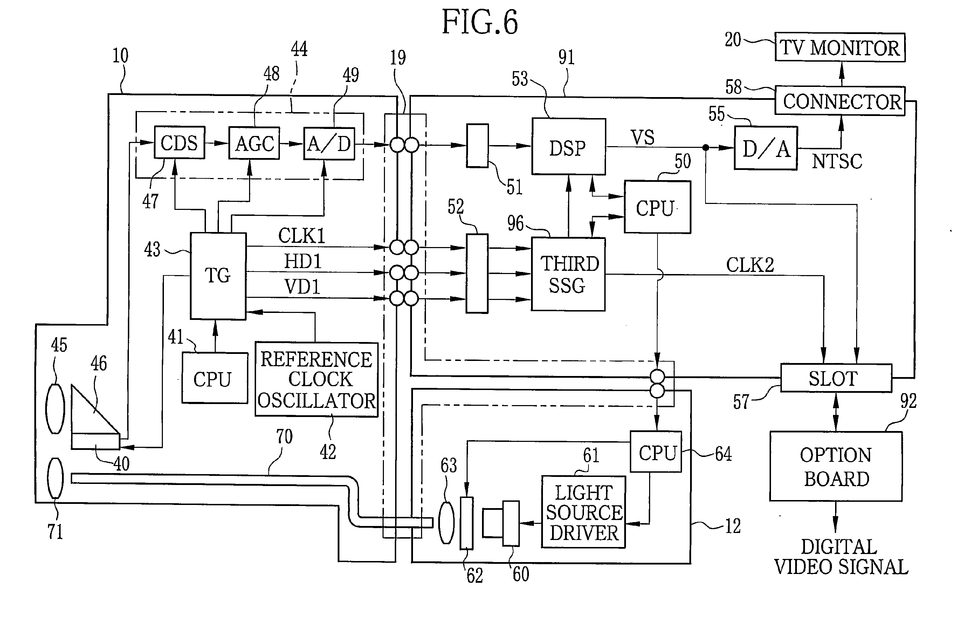

Next, a second embodiment of the present invention will be described. Referring to an endoscope system 90 of Fig. 6, the second clock signal "CLK2" from a third synchronizing signal generator (third SSG) 96 in a processor device 91 and the video signal "VS" are inputted to the slot 57.

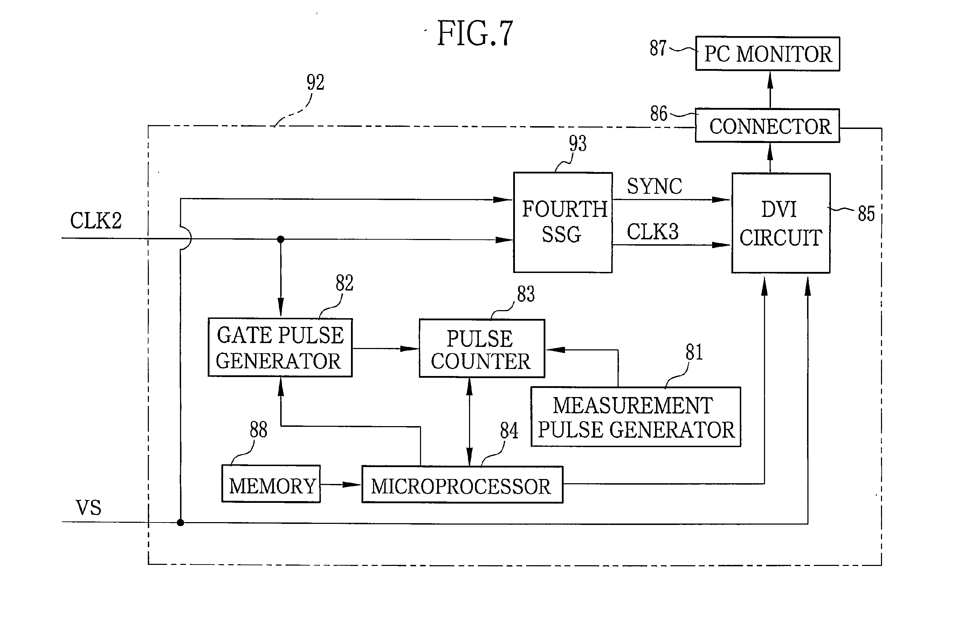

In an option circuit board 92 connected to the slot 57, as shown in Fig. 7, the video signal "VS" and the second clock signal "CLK2" are inputted to a fourth synchronizing signal generator (fourth SSG) 93. The fourth SSG 93 produces a synchronizing signal "SYNC" that synchronizes with the second clock signal "CLK2" from the video signal "VS" and the second clock signal "CLK2".

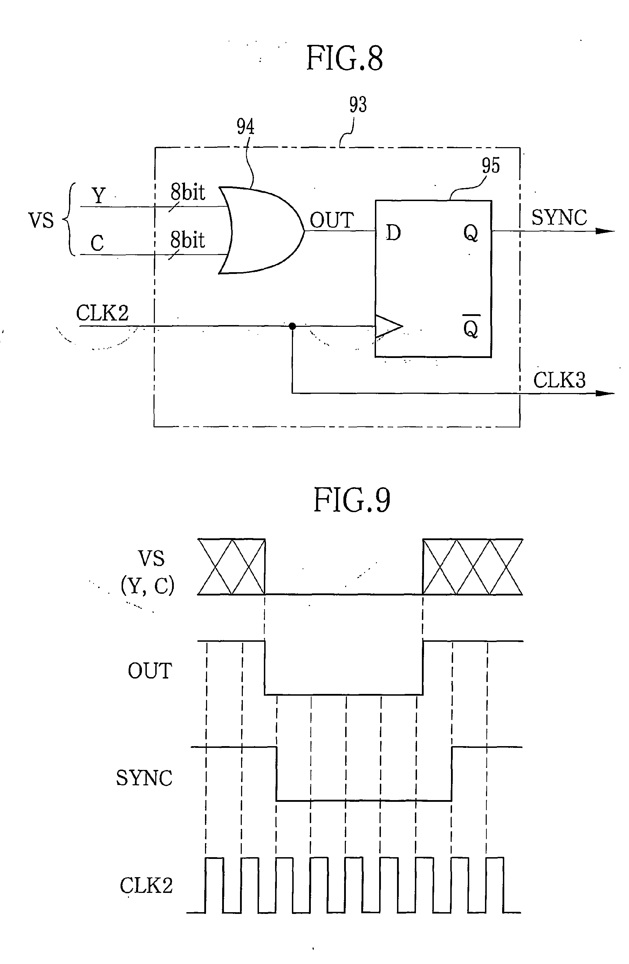

Referring to Fig. 8, the fourth SSG 93 is constituted of an OR circuit 94 and a D flip-flop 95. The video signal "VS" consists of an 8-bit luminance (Y) signal and an 8-bit color difference (C) signal. Eight bits of the Y signal are inputted to the OR circuit 94 in parallel, and eight bits of the C signal are inputted thereto in parallel. The OR circuit 94 outputs a low-level signal when all bits of the Y signal and the C signal are "0", and outputs a high-level signal when at least one bit thereof is "1" (refer to "OUT" of Fig. 9).

An output signal "OUT" of the OR circuit 94 is inputted to a data input terminal "D" of the D flip-flop 95, and the second clock signal "CLK2" is inputted to a clock input terminal thereof. The D flip-flop 95, as shown in Fig. 9, samples the output signal "OUT" at rising edges of the second clock signal "CLK2" and holds data. Accordingly, the synchronizing signal "SYNC" that synchronizes with the second clock signal "CLK2" is outputted from a data output terminal "Q" of the D flip-flop 95.

All bits of the video signal "VS" are "0" in a horizontal or vertical blanking interval of the solid-state image sensor 40. Thus, the synchronizing signal "SYNC" outputted from the D flip-flop 95 coincides with a composite synchronizing signal of the second horizontal synchronizing signal "HD2" and the second vertical synchronizing signal "VD2". The synchronizing signal "SYNC" is inputted to the DVI circuit 85.

As described above, since the endoscope system 90 produces the synchronizing signal "SYNC" from the video signal "VS" and the second clock signal "CLK2", it is possible to further reduce the number of communication lines between the processor device 11 and the option circuit board 56.

The present invention is applicable to an option circuit board that outputs another-format video signal such as a high vision video signal (HDTV signal).

Although the present invention has been fully described by the way of the preferred embodiment thereof with reference to the accompanying drawings, various changes and modifications will be apparent to those having skill in this field. Therefore, unless otherwise these changes and modifications depart from the scope of the present invention, they should be construed as included therein.

## Claims

1. An option circuit board (56, 92) detachably connected to a processor device (11, 91) and having a signal processing circuit (80, 85, 93), said processor device producing a first video signal from an image signal outputted from a solid-state image sensor (40) contained in an electronic endoscope (10), said solid-state image sensor being driven by a clock signal whose frequency corresponds to the number of pixels thereof, said signal processing circuit converting said first video signal into a second video signal in consideration of said number of pixels of said solid-state image sensor to display an image on external equipment (87), said option circuit board comprising:
a measurement pulse generator (81) for generating measurement pulses at constant intervals;
a measurement period setting circuit (82) that sets a period of time during a predetermined number of clocks of said clock signal as a measurement period;
a pulse counter (83) for counting the number of said measurement pulses generated by said measurement pulse generator (81) during said measurement period; and
a judgment circuit (84) for judging said number of pixels from a count value of said pulse counter.

2. The option circuit board (56, 92) as recited in claim 1, wherein said external equipment is a PC monitor (87).

3. The option circuit board (56, 92) as recited in claim 1, wherein said judgment circuit (84) is provided with a memory (88) that stores data indicating relation between said count value and said number of pixels, and said judgment circuit refers to said data in said memory to judge said number of pixels.

4. The option circuit board (56, 92) as recited in claim 1, wherein said signal processing circuit comprises:
a synchronizing signal generator (80, 93) for generating a synchronizing signal; and
a DVI circuit (85) for outputting a DVI-format digital video signal as said second video signal, said DVI-format digital video signal being produced from said first video signal and said synchronizing signal.

5. The option circuit board (56, 92) as recited in claim 4, wherein said synchronizing signal generator (80, 93) corrects deviation in phase between a first horizontal synchronizing signal and a first vertical synchronizing signal, which are outputted from said processor device (11), and then outputs a second horizontal synchronizing signal and a second vertical synchronizing signal as said synchronizing signals.

6. The option circuit board (92) as recited in claim 4, wherein said first video signal includes a luminance signal and a color-difference signal, and said synchronizing signal generator (93) comprises:
an OR circuit (94) to which said luminance signal and said color-difference signal are inputted; and
a flip-flop (95) having a data input terminal, a clock signal input terminal, and an output terminal, an output signal of said OR circuit being inputted to said data input terminal, said clock signal being inputted to said clock signal input terminal, said output terminal outputting said output signal of said OR circuit in synchronization with said clock signal.

7. An endoscope system (2) including an electronic endoscope (10) having a solid-state image sensor (40), a processor device (11, 91) connected to said electronic endoscope, and an option circuit board (56, 92) detachably connected to said processor device, said solid-state image sensor being driven by a clock signal whose frequency corresponds to the number of pixels, said solid-state image sensor capturing an image in a human body cavity and generating an image signal, said endoscope system comprising:
a first signal processing circuit (50, 53-55, 96) provided in said processor device, said first signal processing circuit producing a first video signal from said image signal, said first video signal being sent to an NTSC-format monitor (20) connected to said processor device;
a pixel number judgment section (81-84, 88) provided in said option circuit board, said pixel number judgment section obtaining said number of pixels from a frequency of said clock signal; and
a second signal processing circuit (80, 85, 93) provided in said option circuit board, said second signal processing circuit converting said first video signal into a second video signal in consideration of said number of pixels to display an image on external equipment (87).

8. The endoscope system (2) as recited in claim 7, wherein said external equipment is a PC monitor (87).

9. The endoscope system (2) as recited in claim 7, wherein said pixel number judgment section comprises:
a measurement pulse generator (81) for generating measurement pulses at constant intervals;
a measurement period setting circuit (82) that sets a period of time during a predetermined number of clocks of said clock signal as a measurement period;
a pulse counter (83) for counting the number of said measurement pulses generated by said measurement pulse generator during said measurement period; and
a judgment circuit (84) for judging said number of pixels from a count value of said pulse counter.
